(19) Europäisches Patentamt
European Patent Office
Office européen des brevets

(11) **EP 1 808 441 A1**

(12) **EUROPEAN PATENT APPLICATION**

(43) Date of publication:
**18.07.2007 Bulletin 2007/29**

(51) Int Cl.:
*C07K 14/435* (2006.01)  *C12N 15/12* (2006.01)
*G01N 33/68* (2006.01)

(21) Application number: **06000452.0**

(22) Date of filing: **11.01.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **AXXAM S.p.A.**
**20132 Milano (IT)**

(72) Inventors:
• **Cainarca, Silvia,**
Axxam S.p.A.
**20132 Milan (IT)**

• **Nucci, Cinzia,**
Axxam S.p.A.
**20132 Milan (IT)**
• **Corazza, Sabrina,**
Axxam S.p.A.
**20132 Milan (IT)**
• **Lohmer, Stefan,**
Axxam S.p.A.
**20132 Milan (IT)**

(74) Representative: **Capasso, Olga et al**
**de Simone & Partners SpA**
**Via V.Bellini 20**
**00198 Roma (IT)**

(54) **Luminescent stem cells, transgenic mammals and uses thereof**

(57) It is described a stable recombinant stem cell able to express an apophotoprotein and produce a bioluminescent signal in the presence of a suitable chromophore as substrate in response to intracellular calcium concentration variation; non human transgenic mammal and cell derivatives thereof produced by said cells; methods for identifying agents modulating the differentiation of stem cells towards a specific cell lineage; methods for identifying a ligand able to stimulate a specific cell lineage target; methods for identifying an antagonist to ligand known to stimulate a specific cell lineage target; uses of stable recombinant stem cells for *in vitro* testing of toxicity and/ teratology of a substance.

EP 1 808 441 A1

## Description

The present invention relates to recombinant stem cells stably transfected with a gene encoding for a photoprotein. In particular the invention refers to non human totipotent stem cells, to pluripotent embryonic stem cells, to human and non-human pluripotent tumoral cells, multipotent adult stem cells and progenitors thereof. The recombinant photoprotein stem cell lines are used for different purposes as i.e. in High Throughput Screening, both in a undifferentiated state for identifying agents stimulating or inhibiting the differentiation towards a specific cell lineage, and in the differentiated state for performing screening on endogenously expressed target genes. Moreover photoprotein transfected non human embryonic stem cells are used to generate transgenic animals able to synthesize and express the photoprotein *in vivo.*

## BACKGROUND OF THE INVENTION

Stem cells are unspecialized cells that are able to renew themselves through cell division for long periods (1). Moreover, under certain physiologic or experimental conditions, they can be differentiated into different cell types such as beating cardiomyocytes or insulin-producing cells of the pancreas (2, 3).

Stem cells can be subdivided and classified on the basis of their potency. Totipotent stem cells are produced from the fusion between an egg and a sperm cell. Cells produced by the first few divisions of the fertilized egg cell are also totipotent. These cells can grow into any type of cell. Pluripotent stem cells are the descendants of totipotent cells and can differentiate into any cell type except for totipotent stem cells. Multipotent stem cells can produce only cells of a closely related family of cells (e.g. blood cells such as red blood cells, white blood cells and platelets). Progenitor (sometimes called unipotent) cells can produce only one cell type, but have the property of self-renewal which distinguishes them from non-stem cells (3-6).

Stem cells can also be categorized according to their source, as either adult or embryonic. Adult stem cells are undifferentiated cells found among differentiated cells of a specific tissue and are mostly multipotent, capable of producing several but limited numbers of cell types. They comprise also newborne- umbilical cord-derived stem cells. They are also called somatic stem cells, or tissue stem cells, and are found in differentiated tissues in which, in a controlled manner, they differentiate and/or divide to produce all the specialized cell types of the tissue from which they originate (7-9).

Embryonic stem cells have the potential of becoming all types of specialized cells including germ cells (pluripotency). They have the capability of proliferating indefinitely in culture, under conditions that allow their proliferation without differentiation (3). Three types of pluripotent embryonic stem cells have been discovered up to now from rodents and humans (10):

- Embryonic Stem cells (ES) which derive from the inner cell mass of the pre-implantation blastocyst stage embryo. They have a normal karyotype. Several types of mouse and human embryonic stem cells are known and established, like for example the mouse ES TBV2, R1, D3 cells (11-13, 63).
- Embryonic Carcinoma cells (EC) which derive from teratocarcinomas. Teratocarcinomas are gonadal tumors containing pluripotent stem cells present within a wide array of tissues derived from the three primary germ layers (endo-, meso-, and ecto-derm). The most used ones are the mouse EC P 19 cell lines. These cells don't have a normal karyotype (14-18).
- Embryonic Germ cells (EG) which derive from cultured Primordial Germ Cells of the foetal genital ridge (PGCs). They have a normal karyotype but are already genetically imprinted (19-22).

ES and EG cells can be injected into blastocysts of recipient mice giving rise to chimeric animals. In chimeric mice these pluripotent cells can contribute to every cell type, including the germline (20, 21, 23, 24). In contrast, murine EC cells introduced into embryos colonize most embryonic lineages, but generally do not colonize the germline, with one experimental exception (25-27). The inability of EC cells to form functional gametes most likely reflects their abnormal karyotype (28).

Stem cells are a very powerful tool for High Throughput Screening (HTS) technologies since they can be cultured and expanded *in vitro* for long periods, maintaining the self-renewal property, and they can undergo miniaturization. They allow the use of selectable and inducible markers for the preparation of a pure population ES cells. The technology of gene targeting/homologous recombination allows the Knock Out (KO) or Knock In (KI) of specific genes. Furthermore embryonic stem cells can differentiate into any cell type resembling primary cells (since they are non tumoral cells). In this way they offer a natural environment for the targets, they can address complex targets (like multi-subunit ion channels), that are regulated and expressed in a native way. This is a very important improvement since usually in HTS screening the cell-based assays are set up using tumoral cell lines and it is known that this tumoral environment can alter the physiological cell conditions.

The use of pluripotent embryonic stem cells has acquired a fundamental role in the pharmaceutical field (29). For example for target evaluation, since understanding gene function in drug discovery is fundamental for success, murine ES cells represent a more rapid and less expensive tool compared to KO mice. In addition in case of a lethal KO, the use of ES cells could be very helpful for gene function evaluation.

Stem cells have also been used for the Embryonic Stem cell Test (EST) which was positively evaluated by the EVCAM study (European Centre for the Validation of Alternative Methods). This is a test of toxicology and teratology for drugs on the cellular and tissue differentiation generated from the 3 germ lineages (endo-, meso-, and ecto-derm). Stem cells are also very important for the analysis of the drugs secondary effects on the chronotropic activity on pulsing cardiomyocytes obtained by differentiation of pluripotent stem cells. This kind of test can reduce the number of animals used for toxicological studies. For example in the European Union up to 30000 chemicals that are currently on the market have to be re-evaluated in the next 10 years. This means the use of about 10 million animals. The creation of *in vitro* tests like the EST can be crucial in this sense and can also allow the testing of more chemicals in less time than conventional whole-animal experiments (30, 31, 32).

Bioluminescence is the phenomenon by which visible light is emitted by living organisms or by a substance derived from them through a variety of chemiluminescent reaction systems. Bioluminescence reactions require three major components: a luciferin (substrate), a luciferase (enzyme) and molecular oxygen. However, other components may also be required in some reactions, including cations ($Ca^{++}$ and $Mg^{++}$) and cofactors (ATP, NAD(P)H). Luciferases are enzymes that catalyse the oxidation of a substrate, luciferin, and produce an unstable intermediate. Light is emitted when the unstable intermediate decays to its ground state, generating oxyluciferin. There are many different unrelated types of luciferin, although many species from at least seven phyla use the same luciferin, known as coelenterazine. In some animals (e.g. jellyfish) the luciferin/luciferase system can be extracted in the form of a stable "photoprotein" which emits light upon calcium binding. Photoproteins differ from luciferases in that they are stabilized oxygenated intermediate complexes of luciferase and luciferin. Photoproteins are present in many marine coelenterates and allow these organisms to emit light for a variety of purposes including breeding, feeding and defense (33). There are many luminescent organisms, but only seven photoproteins, namely Thalassicolin (34,35), Aequorin (36,37,38), Mitrocomin (syn. with Halistaurin) (39,40), Clytin (syn. with Phialidin) (40, 41), Obelin (34,38,42,43), Mnemiopsin (44,45) and Berovin (44,45) have been isolated so far. All these proteins are complexes formed by an apoprotein, an imidazopyrazine chromophore (i.e., coelenterazine) and oxygen. Their amino acid sequences are highly conserved, especially in the region containing the three calcium binding sites (EF-hand structures). The term "photoprotein" identifies the coelenterazine-bound polypeptide, which is capable of luminescence, while "apophotoprotein" is used to indicate the protein without coelenterazine.

The most studied photoproteins are Aequorin, isolated from *Aequorea victoria* (46) and Obelin, isolated, from *Obelia longissima* (47). The photoprotein may be regenerated from the apophotoprotein by incubation with coelenterazine, molecular oxygen, EDTA and 2-mercaptoethanol or dithiothreitol. Since coelenterazine is the common luminescent substrate used by the photoproteins Aequorin, Mitrocomin, Clytin and Obelin, the light-emitting reaction is likely the same in these four photoproteins (48,49,50,51).

The study of cellular events and their regulation requires sensitive, non invasive analytic methods. Photoproteins and in general the use of bioluminescence are excellent reporter systems as they have virtually no background in contrast to fluorescence systems.

Photoproteins are widely used in cell culture systems as reporter genes to monitor the cellular events associated with signal transduction and gene expression (33,34,46). Photoproteins are expressed in mammalian cells to monitor calcium changes in response to different stimuli. Intracellular calcium concentrations can be measured by adding the cofactor coelenterazine to mammalian cells expressing the photoprotein and detecting photon emission, which is indicative of intracellular calcium concentration. The use of cells which express both a photoprotein and a receptor involved in the modulation of intracellular calcium concentration provides a valid system for the screening of compounds for their effects on the release of intracellular calcium.

High throughput screening assays are often designed using a photoprotein as a reporter system. The sensitivity of the system as well as its high signal to noise ratio allow the use of small assay-volumes.

Calcium flux assays are commonly carried out in HTS format utilizing optical screening apparatus suited for the simultaneous analysis of a high number of samples and equipped with a luminescence imaging systems.

However, calcium concentration variation can also be detected using fluorescent calcium dyes like for example Fluo3, Fluo4, Fura2 and Calcium dyes (Molecular Devices and Molecular Probes) using fluorimetric instruments like FLIPR® (Fluorometric Imaging Plate Reader, Molecular Devices Corporation, Sunnyvale, CA, USA), one of the most used instruments in HTS assays. The apparatus is equipped with an optical detection device that allows signal isolation on a cell-monolayer, thereby enhancing sensitivity for cell-based assays.

The most recent FLIPR® system versions have been made suitable also for luminescence assays, even if with lower sensitivity compared to CCD camera-based equipments. To overcome the lower luminescence sensitivity of this system, photoproteins with enhanced light emission are highly advantageous.

The authors of the instant invention developed a system based on stem cells stably transfected with a photoprotein coding sequence, by means of appropriate vectors. The transfected stem cell is used directly in different screening methods or used to generate non human transgenic mammals able to express the photoprotein coding sequence *in vivo*.

## DISCLOSURE OF THE INVENTION

It is an object of the invention a stable recombinant stem cell able to express an apophotoprotein and produce a bioluminescent signal in the presence of a suitable chromophore substrate in response to intracellular calcium concentration variation.

Stem cell is intended a totipotent and/or pluripotent non human cell; or a human or non-human pluripotent tumoral cell, or a multipotent cell or a progenitor thereof, being of embryonic or of adult origin. In particular preferred stem cells are mouse embryonic stem cells, preferably ES TBV2 (63) cells and the mouse embryonic carcinoma cell line, P19 (26-28, 61). P19 cell line can be of some advantage because it can be cultured in the undifferentiated state without the need of LIF (Leukemia Inhibitory Factor) and/or feeder cell layers.

Apophotoprotein is intended any apophotoprotein, natural or recombinant or synthetic. The apophotoprotein may be a natural or a mutagenized mutant, also having an improved luminescent activity and/or calcium sensibility, and a chimeric protein derived from two different natural apophotoproteins, also further modified by deletion, addition or substitution of one or more amino acid residues, provided that the activity profile of the photoprotein, in terms of light-emission and calcium-responsiveness, is maintained or increased. Apophotoprotein sequences may also be optimized for mammalian codon usage and/or fused to mitochondrial target sequences (52,53,54). Photoproteins with enhanced bioluminescence are already disclosed in the prior art, i.e the photoprotein Photina™ (described in EP 1413584, and herein reported as SEQ ID No. 1) obtained by chimerization of the protein Obelin with a region of the Clytin protein.

Photoproteins with enhanced bioluminescence may also derive from mutagenesis, as the Clytin sequence (GenBank accession number Q08121) mutagenised in the following position $Gly_{142} \rightarrow Cys$; or the Clytin sequence (GenBank accession number Q08121) mutagenised in the following 12 positions: $Gly_{58} \rightarrow Glu$, $Asp_{69} \rightarrow Val$, $Ala_{70} \rightarrow Cys$, $Lys_{76} \rightarrow Arg$, $Lys_{77} \rightarrow Gly$, $Ile_{78} \rightarrow Cys$, $Asp_{81} \rightarrow Glu$, $Val_{86} \rightarrow Ile$, $Glu_{87} \rightarrow Ala$, $Ala_{90} \rightarrow Gln$, $Val_{92} \rightarrow Leu$, and $Glu_{97} \rightarrow Gln$.

Stable recombination may be achieved with standard transfection methods known to the skilled in the art as but not limited to electroporation, PEG $Ca^{++}$ precipitation, Cationic Lipid methods, etc.

Advantageously the stable recombinant stem cell may be differentiated into a specific cell lineage to get expression of at least one specific cell lineage target, preferably the muscle heart cell lineage, alternatively the neuronal lineage, alternatively the mesenchimal cell lineage.

Another object of the invention is a non human transgenic mammal able to express a photoprotein gene and produce a bioluminescent signal in response to intracellular calcium concentration variation.

Preferably the apophotoprotein gene expression and/or the production of a bioluminescent signal in response to intracellular calcium concentration variation is ubiquitous; alternatively is organ-, tissue-, cell- or development stage-specific.

The invention advantageously provides methods for the identification and/or testing of compounds for many applications, i.e. therapeutic, diagnostic applications. In the context of the invention a compound library is a collection, either synthetic or recombinant, of compounds to be tested or identified.

Another object of the invention is a method for identifying agents stimulating the differentiation of stem cells towards a specific cell lineage comprising the steps of:

a) providing stable recombinant stem cells according to the invention at an undifferentiated stage;
b) exposing said cells to a compound library comprising putative inducing differentiation agents to get expression of at least one specific cell lineage target;
c) loading cells with a suitable chromophore as substrate;
d) stimulating said specific cell lineage target by a ligand so that a variation of intracellular $Ca^{++}$ is obtained;
e) detecting photoprotein's bioluminescence.

Preferably the specific cell lineage is the muscle heart cell lineage or the neuronal lineage.

In a preferred embodiment the method is performed by High Throughput Screening.

Another object of the invention is a method for identifying agents inhibiting the differentiation of stem cells towards a specific cell lineage comprising the steps of:

a) providing stable recombinant stem cells according to the invention at an undifferentiated stage;
b) exposing said cells to a compound library comprising putative inhibiting differentiation agents;
c) exposing said cells to a known inducing differentiation agent to get expression of at least one specific cell lineage target;
d) loading cells with a suitable chromophore as substrate;

e) stimulating said specific cell lineage target by a ligand so that a variation of intracellular $Ca^{++}$ is obtained;

f) detecting photoprotein's bioluminescence.

Preferably the specific cell lineage is the muscle heart cell lineage or the neuronal lineage.

In a preferred embodiment the method is performed by High Throughput Screening.

Another object of the invention is a method for identifying a ligand able to stimulate a target so that a variation of intracellular $Ca^{++}$ is obtained.

Stem cells differentiated into a specific cell lineage resemble primary cells; advantageously the methods allow to study and modulate target receptors, transporters and channels, often made of complex multi-subunits, endogenously expressed by the cells, in the most natural cellular context.

The use of stem cells in HTS gives a more accurate and physiological evaluation of targets, is more reproducible and therefore is more reliable for the drug discovery process.

The method comprises the steps of:

a) providing stable recombinant stem cells according to the invention;

b) differentiating said cells into a specific cell lineage to get the expression of the target;

c) loading cells with a suitable chromophore as substrate;

d) contacting cells with a compound library comprising putative ligands for said target;

e) detecting the photoprotein's bioluminescence.

Preferably the specific cell lineage is the muscle heart cell lineage or the neuronal lineage.

In a preferred embodiment the method is performed by High Throughput Screening.

Another object of the invention is a method for identifying antagonists to a target, so that a variation of intracellular $Ca^{++}$ is obtained, comprising the steps of:

a) providing stable recombinant stem cells according to the invention;

b) differentiating said cells into a specific cell lineage to get expression of said specific target;

c) loading cells with a suitable chromophore as substrate;

d) contacting cells with a compound library comprising putative antagonists for said target;

e) contacting cells with a ligand able to stimulate the said target;

f) detecting the photoprotein's bioluminescence variation.

Preferably the specific cell lineage is the muscle heart cell lineage or the neuronal lineage.

In a preferred embodiment the method is performed by High Throughput Screening.

Another object of the invention is the use of the stable recombinant stem cells, either undifferentiated or differentiated, for *in vitro* testing of toxicity and/ teratology of a substance. For example the Embryonic Stem Cell Test (EST) *in vitro* system may allow to test toxic and/or teratogenic effects of test chemicals on beating cardiomyocytes in embryoid bodies compared to cytotoxic effects on undifferentiated murine ES cells and differentiated 3T3 fibroblasts, being altered cardiogenesis a valid indicator of the embryotoxic potential of chemicals. These methods can also be adapted and used in HTS systems (30-32).

As mentioned above, the methods of the invention are preferably carried out in a High Throughput Format, i.e. 96, 384 or 1536 Micro-Titer-Plates (MTP) utilizing an optical screening tool or apparatus suited for multi-sample analysis, such as a luminescence imaging system with a CCD camera-based luminometer detector for high and ultra high throughput applications, or with the Fluorometric Imaging Plate Reader (FLIPR®).

Typically, stem cells are stably transfected with an expression vector containing a photoprotein encoding sequence. The positive clones are selected and plated in a suitable medium, cultured cells are loaded with the coelenterazine substrate and the assay is started by adding the test molecule or stimulus. The produced luminescence is read by many suitable detection systems optimized for HTS screening, which can detect luminescence by the use of a CCD camera-based luminometer or other luminometric devices. The photoprotein-expressing cells are plated in microplate wells, which, after addition of the test molecule/stimulus, are read with signal recording devices.

High throughput screening assays set up with a photoprotein-based reporter system show improved sensitivity and signal-to-noise ratio compared to fluorescence-based systems. Stem cells or differentiated derivatives expressing a photoprotein produce an intense bioluminescence in response to calcium stimulation and are useful for studying endogenous targets of interest.

This method of screening for therapeutically active molecules in the most relevant and accurate cellular context is advantageous for the development of new drugs.

The study of cellular events and their regulation requires sensitive, non invasive analytic methods. Photoproteins and in general bioluminescence are often used as effective reporter systems.

The advantages of using luminescent photoprotein assays over the fluorescent methods for HTS screening are many:

- the high calcium sensitivity of photoproteins, in the 1-10 $\mu$M physiological range, allows the detection even in the case of small calcium movements;
- photoproteins have the possibility of being targeted to specific cell compartments, such as the mitochondria, in order to detect calcium release from internal stores in a more precise way (52-54);
- photoprotein-based assays have virtually no background if compared with the high fluorescent background signal of all the calcium sensitive fluorescent dyes. This is reflected in a larger linear dynamic range of luminescent versus fluorescence response;
- the use of photoprotein-based assays is preferable in HTS because it avoids the problem of compound auto-fluorescence that often leads to a higher false-positive hit-rate in fluorescence based assays;
- the substrate used in photoprotein based luminescence assays (coelenterazine) has no toxicity to cells and is not subject to efflux, whereas often calcium sensitive dyes used in fluorescence assays are cytotoxic;
- the possibility of using cells in suspension is an alternative assay process;
- no requirement for cell washing before the analysis and low cost per test point.

Photoprotein-based assays have advantages also over the classical luciferase based assay, since the light signal is generated immediately after the compound addition during screening. In fact photoproteins are constitutively expressed in cell lines and ready to react with compounds, whereas in classical luciferase based assays the incubation time of compounds with cells is longer due to the time needed for induced synthesis of the luciferase gene.

The invention will be described in more detail in the following experimental section by reference to the following figures:

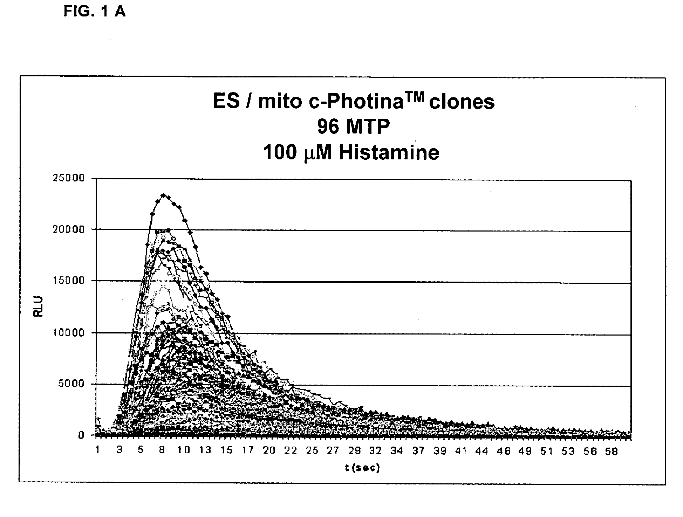

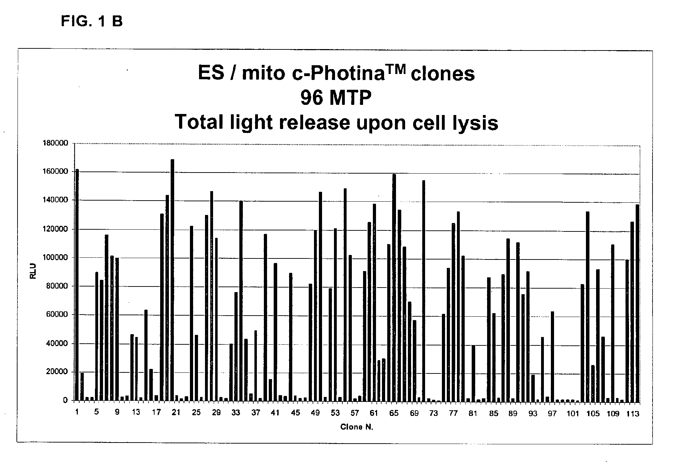

Fig. 1 Clone pool analysis of the 114 neomycin resistant clones. A. Histamine response kinetics (100 $\mu$M) was measured at CCD camera-based luminometer and recorded as RLU (Relative Luminescence Units) values. The experiments were performed in 96 MTP 24 h after cell seeding. CCD camera-based luminometer conditions: high sensitivity, for 60 seconds. **B**. Total photoprotein residual activity was measured after TRITON X-100 cell lysis. CCD camera-based luminometer conditions: low sensitivity, reading time 5 seconds.

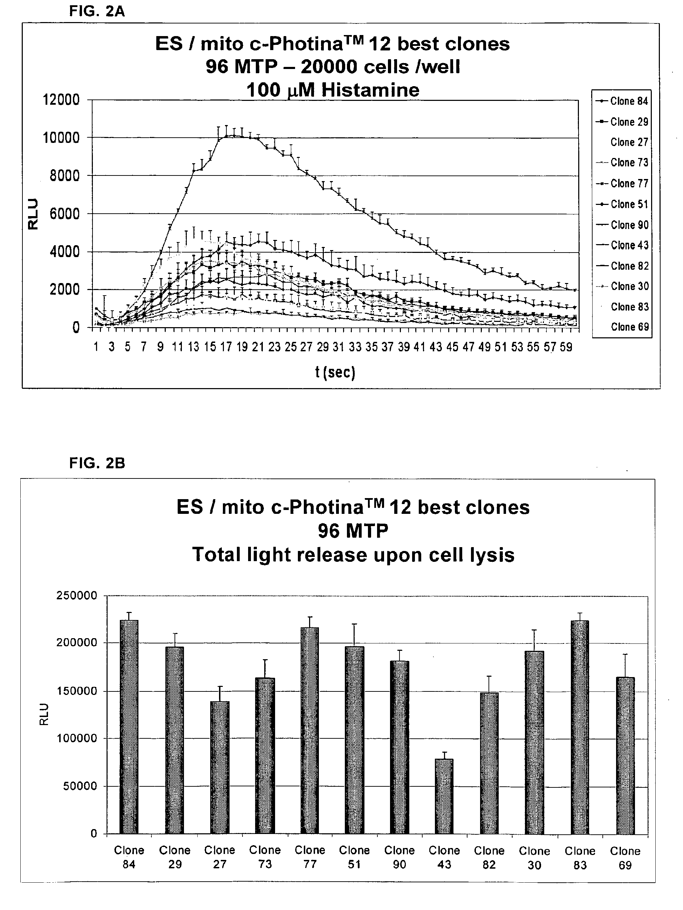

**Fig. 2** Analysis of the 12 ES / mito c-Photina™ best clones. **A**. Histamine response (100 $\mu$M) of the 12 best mito c-Photina™ ES clones was measured in 96 MTP 24 h after seeding 20000 cells/well. CCD camera-based luminometer conditions: high sensitivity, reading time 60 seconds. **B**. Total photoprotein residual activity of the 12 best mito c-Photina™ ES clones was measured after TRITON X-100 cell lysis. CCD camera-based luminometer conditions: low sensitivity, reading time 5 seconds.

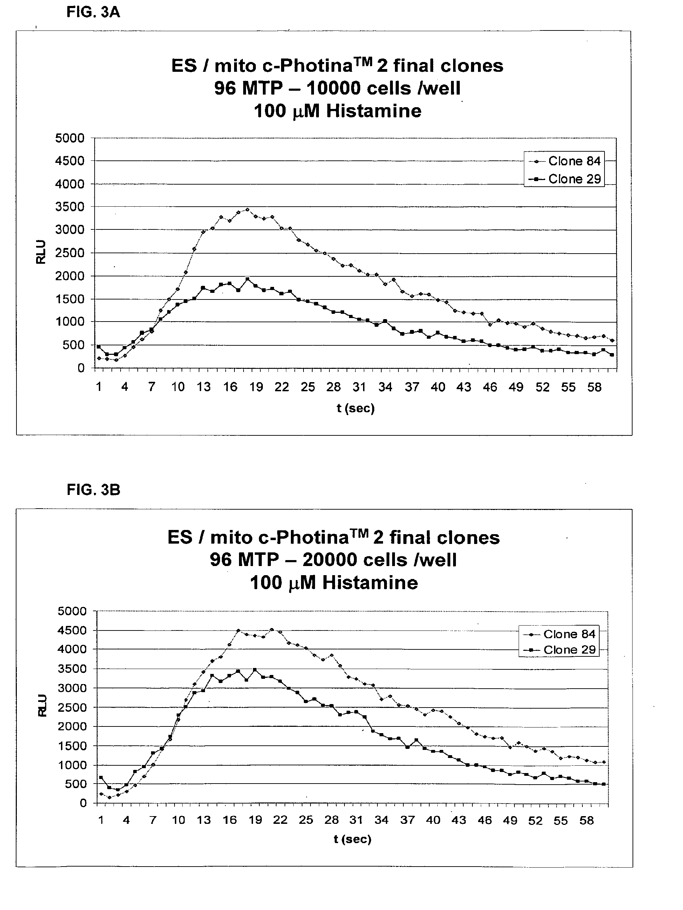

**Fig. 3** Analysis of the 2 ES / mito c-Photina™ final clones. **A**. Histamine response (100 $\mu$M) was measured in 96 MTP 24 h after seeding 10000 cells/well. CCD camera-based luminometer conditions: high sensitivity, reading time 60 seconds. **B**. Histamine response (100 $\mu$M) was measured in 96 MTP 24 h after seeding 20000 cells/well. CCD camera-based luminometer conditions: high sensitivity, reading time 60 seconds.

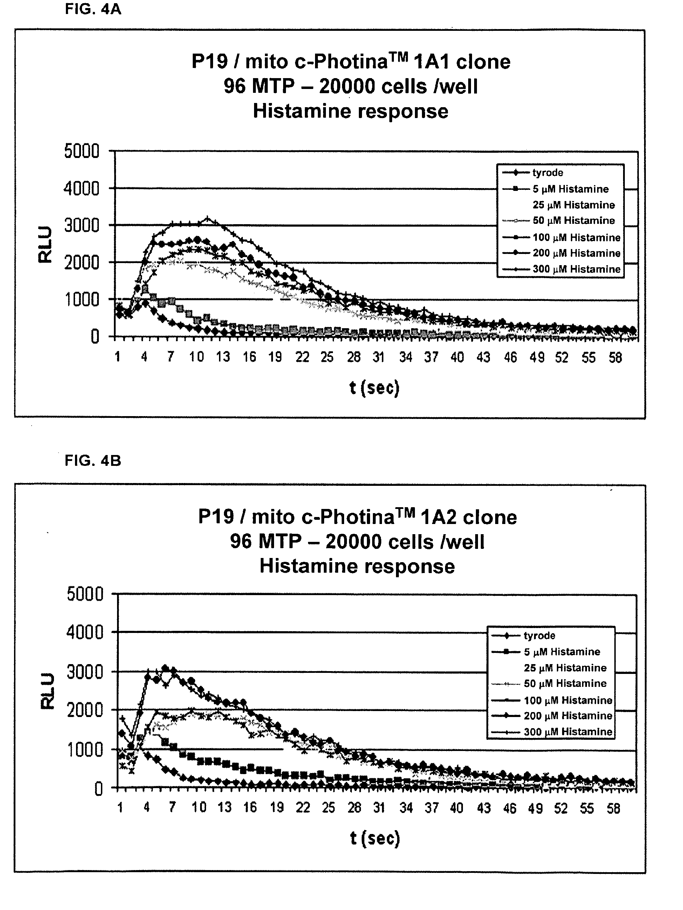

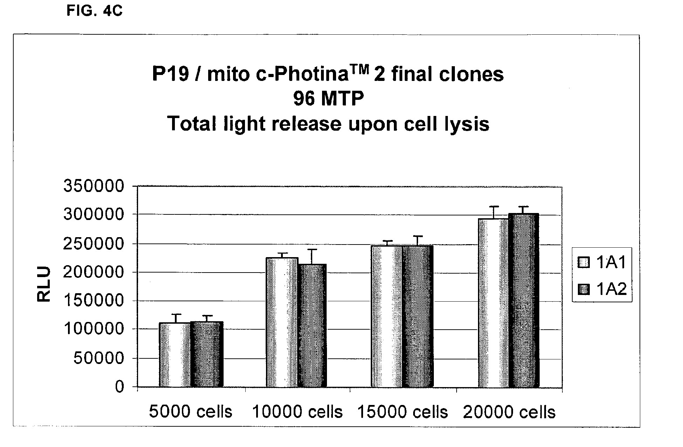

**Fig. 4** Analysis of the P19 / mito c-Photina™ 2 final clones. **A**. Histamine response of the P19 / mito c-Photina™ 1A1 clone was measured in 96 MTP 24 h after seeding 20000 cells/well. CCD camera-based luminometer conditions: high sensitivity, reading time 60 seconds. **B**. Histamine response of the P19 / mito c-Photina™ 1A2 clone was measured in 96 MTP 24 h after seeding 20000 cells/well. CCD camera-based luminometer conditions: high sensitivity, reading time 60 seconds. **C**. Total photoprotein residual activity was measured after TRITON X-100 cell lysis. CCD camera-based luminometer conditions: low sensitivity, reading time 30 seconds.

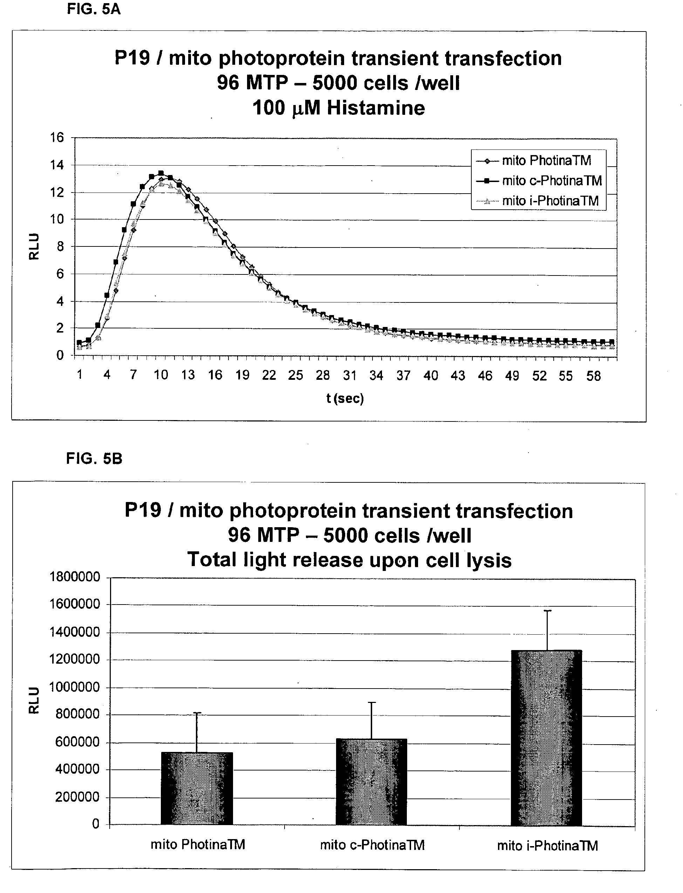

**Fig. 5** Transient transfection of different photoproteins (mito Photina™, mito c-Photina™, mito i-Photina™) in P19 cells. **A**. 100 $\mu$M Histamine response was measured at Luminoskan luminometer. Reading time 60 seconds. **B**. Total light emission was measured after cell lysis with TRITON-X100 at Berthold luminometer. Reading time 3 seconds.

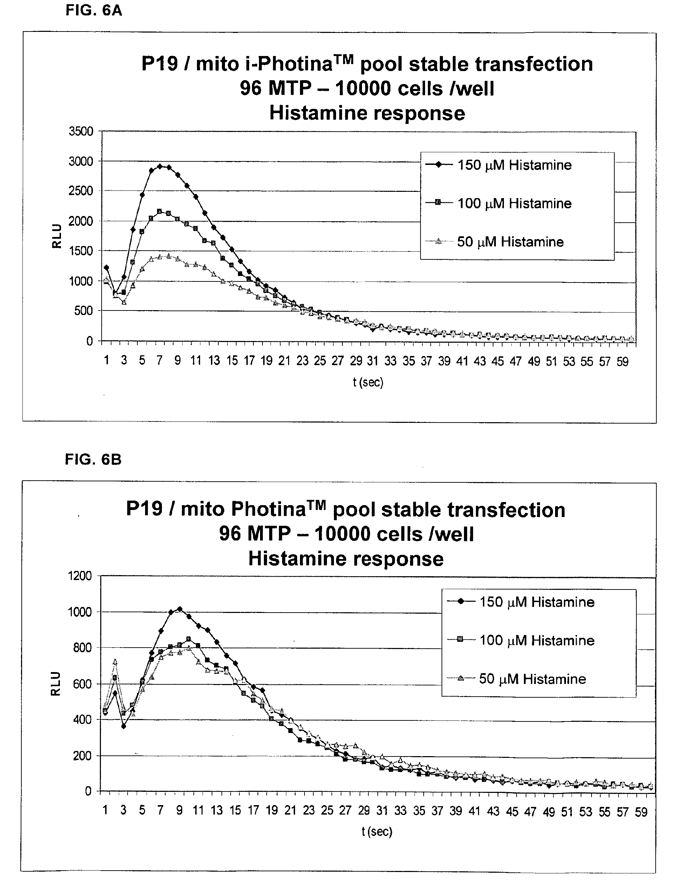

**Fig. 6** Analysis of the pool of mito i-Photina™ (**A**.), and mito Photina™ (**B**.) stably transfected P19 cells. 50, 100 and 150 $\mu$M Histamine dose-response in 96 MTP was measured at 24 h after cell seeding of 10000 cells/well. CCD camera-based luminometer conditions: high sensitivity, reading time 60 seconds.. C. Total light emission was measured after cell lysis with TRITON-X100 in 96 MTP 24 h after seeding 10000 and 20000 cells/well. CCD camera-based luminometer conditions: low sensitivity, reading time 30 seconds.

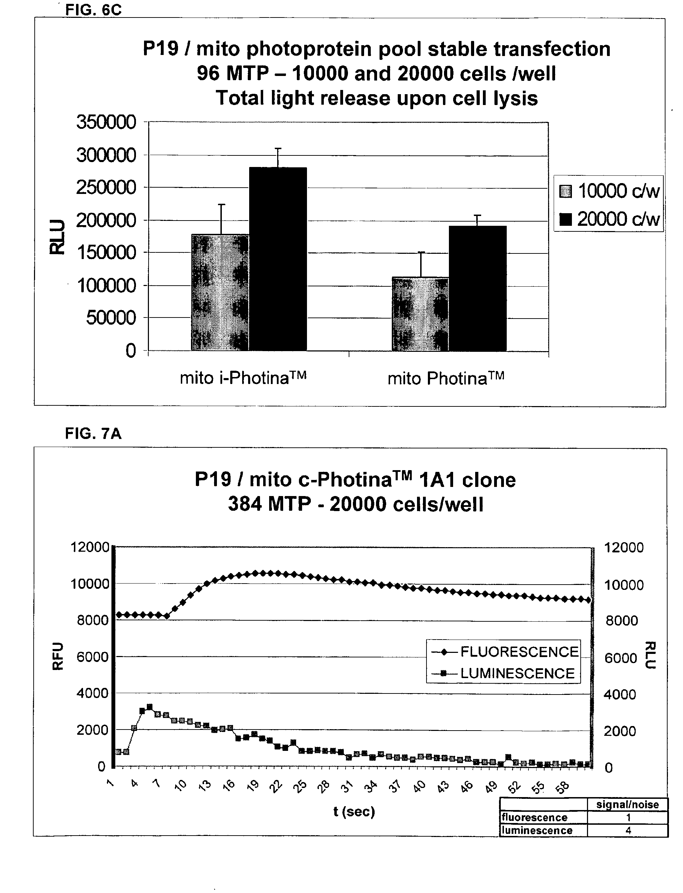

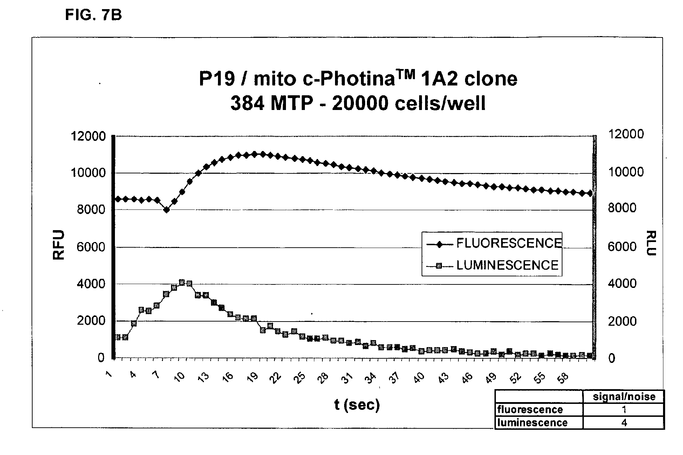

**Fig. 7** Comparison at FLIPR and CCD camera-based luminometer of the P19 mito c-Photina™ 2 final clones. Histamine response of the P19 / mito c-Photina™ 1A1 clone (**A**.) and of the P19 / mito c-Photina™ 1A2 clone (**B**.) was measured in 384 MTP 24 h after seeding 20000 cells/well. For CCD camera-based luminometer analysis medium was replaced 4 h before reading with 10 $\mu$M coelenterazine solution at 37˚C (CCD camera-based luminometer conditions: high sensitivity, reading time 60 seconds). For FLIPR® analysis the medium was replaced 30 min before reading with Calcium 3 Assay kit 0.5X for 30 min at 37˚C (FLIPR[384] settings: Exp. Time: 0.2 sec; injection speed: 20 $\mu$l/sec; injection height: 50 $\mu$l; reading time: 60 seconds. For both measurements the final concentration of Histamine was 100 $\mu$M. The signal to noise ratio was calculated and reported both for luminometer (expressed in RLU: Relative Luminescence Units) and fluorometer (expressed in RFU: Relative Fluorescence Units).

## MATERIALS AND METHODS

### Photoprotein description

### Photina™

The chimeric photoprotein Photina™ is described in Patent EP 1413584, herein reported as SEQ ID No. 1:

```
Met Ser Ser Lys Tyr Ala Val Lys Leu Lys Thr Asp Phe Asp
Asn Pro Arg Trp Ile Lys Arg His Lys His Met Phe Asp Phe
Leu Asp Ile Asn Gly Asn Gly Lys Ile Thr Leu Asp Glu Ile
Val Ser Lys Ala Ser Asp Asp Ile Cys Ala Lys Leu Gly Ala
Thr Pro Glu Gln Thr Lys Arg His Gln Asp Ala Val Glu Ala
Phe Phe Lys Lys Ile Gly Met Asp Tyr Gly Lys Glu Val Glu
Phe Pro Ala Phe Val Asp Gly Trp Lys Glu Leu Ala Thr Ser
Glu Leu Lys Lys Trp Ala Arg Asn Glu Pro Thr Leu Ile Arg
Glu Trp Gly Asp Ala Val Phe Asp Ile Phe Asp Lys Asp Gly
Ser Gly Thr Ile Thr Leu Asp Glu Trp Lys Ala Tyr Gly Lys
Ile Ser Gly Ile Ser Pro Ser Gln Glu Asp Cys Glu Ala Thr


Phe Arg His Cys Asp Leu Asp Asn Ser Gly Asp Leu Asp Val
Asp Glu Met Thr Arg Gln His Leu Gly Phe Trp Tyr Thr Leu
Asp Pro Glu Ala Asp Gly Leu Tyr Gly Asn Gly Val Pro
```

### i-Photina™

i-Photina™ (Patent Application EP05005390.9) is obtained by mutagenesis $Gly_{142} \to Cys$ of the Clytin photoprotein (GenBank accession number Q08121).

### c-Photina™

The c-Photina™ (Patent Application EP06000171) is obtained mutating the Clytin sequence (GenBank accession number Q08121) in the following 12 positions: $Gly_{58} \to Glu$, $Asp_{69} \to Val$, $Ala_{70} \to Cys$, $Lys_{76} \to Arg$, $Lys_{77} \to Gly$, $Ile_{78} \to Cys$, $Asp_{81} \to Glu$, $Val_{86} \to Ile$, $Glu_{87} \to Ala$, $Ala_{90} \to Gln$, $Val_{92} \to Leu$, and $Glu_{97} \to Gln$.

### Photoprotein optimization for expression in mammalian cells

The codon usage of the *c-Photina*™ and *i-Photina*™ genes were adapted to the codon bias of highly expressed mammalian genes. In addition regions of very high (>80%) or very low (<30%) GC content have been avoided where possible.
For efficient translation initiation the Kozak-consensus sequence was introduced upstream of the start codon.

Two STOP codons were added to ensure efficient termination.

**Cloning procedure**

The genes were cloned in the pcDNA3.1+ vector (Invitrogen) with or without the mitochondrial tag (mito) to obtain pcDNA3 mito c-Photina™. For the mitochondrial targeting (52-54) the human Cytocrome c oxydase, subunit VIII, signal sequence was used:

$$5^{'}\text{-ATGTCCGTCCTGACGCCGCTGCTGCTGCGGGGCTT}$$
$$\text{GACAGGCTCGGCCCGGCGGCTCCCAGTGCCGCGCGC}$$
$$\text{CAAGATCCATTCGTTGGGATCCGCCACC-3' (SEQ ID No. 2).}$$

The construct obtained was verified by full-length dideoxy sequencing.

**ES Cell Culture**

ES cells were cultured using standard methods (55, 56).

ES Culture medium, seeding and incubation:

TBV2 (129S2/SvPas) embryonic stem cells (63) are cultured with 15% Foetal Bovine Serum, FBS (ES qualified, Invitrogen, Cat. N. 16141079) DMEM Dulbecco's Modified Eagles Medium, high glucose, without NaPiruvate (Invitrogen, Cat. N. 10313021), 100 $\mu$M $\beta$-Mercaptoethanol (Invitrogen, Cat. N. 31350010), 2 mM Glutamine (Invitrogen, Cat. N. 25030024), 1000U/ml Leukemia Inhibitory Factor, LIF (Prodotti Gianni, Cat. N. ESG1107) at 37˚C, 5% $CO_2$.
Primary Mouse Embryonic Fibroblasts (MEF) cells are cultured 10% Foetal Bovine Serum, FBS (Celbio, Cat. N. CHA11152) DMEM Dulbecco's Modified Eagles Medium, high glucose (Invitrogen, Cat. N. 10313021), 1 mM Sodium Pyruvate (Invitrogen, Cat. N.11360039) non essential aminoacids (Invitrogen, Cat. N. 11140-035), 2 mM Glutamine (Invitrogen, Cat. N. 25030024) at 37˚C, 5% $CO_2$.

Stable transfection

DNA constructs corresponding to the photoproteins were transfected using electroporation methods. 30-40 $\mu$g of the *mito c-Photina*™ and *i-Photina*™ DNA, linearized with BglII (New England Biolabs), were transfected using $7\times10^6$ ES cells (electroporation condition: 500$\mu$F, 0.24 kV, BioRad gene pulser) and incubate on ice for 10-20 minutes. The cell suspension was diluted in ES cell medium containing LIF and transferred on gelatinized 100mm-diameter plates. After approximately 48 hours selection was started using ES media containing 200 $\mu$g/ml G148 (Geneticin, SIGMA, Cat. N. G5013).
Colonies were generally ready for picking 8-9 days after electroporation.

Clone Selection Process:

1. 114 ES pcDNA3/ mito c-Photina™ clones were picked.
2. 24 h and 48 h after seeding, the transfected cells were plated in 2x96MTP white plates in ES medium with LIF.
3. Medium was replaced with 50$\mu$l/well of tyrode (130 mM NaCl, 5 mM KCl, 2 mM $CaCl_2$, 1 mM $MgCl_2$, 5 mM $NaHCO_3$ and 20 mM HEPES, pH 7.4, 2 mM $Ca^{2+}$) and coelenterazine 10 $\mu$M (Pharma Tech International).
4. Positive clones were selected evaluating:

- Response to 100 $\mu$M Histamine (Sigma, H7125-5G). CCD camera-based luminometer conditions: high sensitivity, reading time 60 seconds.
- Residual total photoprotein activity measured after TRITON X-100 cell lysis. CCD camera-based luminometer conditions: low sensitivity, reading time 5 seconds. 12 clones were chosen and expanded and retested at counted cells 10000-20000 c/w in 96 MTP and 2500 c/w in 384 MTP.
- Response to 100 $\mu$M Histamine. CCD camera-based luminometer conditions: high sensitivity, for 60 seconds.

- Residual total photoprotein activity measured after TRITON X-100 lysis. CCD camera-based luminometer conditions: low sensitivity, for 5 seconds.

**DNA extraction**

DNA from ES cells plated on gelatine coated dishes was extracted with standard Proteinase K digestion and phenol-clorophorm-isopropanol extraction method (59). **Determination of the number of insertions by Quantitative PCR**

QPCR (Quantitative Polymerase Chain Reaction) was performed using approximately 3 ng of DNA per reaction with the "Platinum® SYBR Green® QPCR SuperMix UDG" protocol (60, Invitrogen). The primers used were designed using the Primer Express® Software v2.0 (Applied Biosystems), on *c-Photina*™ (CPH) and *neomycin* (neo) genes to detect the plasmid used in the transfections, and specific to the *gusB* gene to detect the genomic DNA:

CPH-for: CACCAAGTGTGCGTGGAGG (SEQ ID No. 3);
CPH-rev: GCGATCTCCTTGCCGTACTC (SEQ ID No. 4);
neo-for: CACGTACTCGGATGGAAGCC (SEQ ID No. 5);
neo-rev: CCCTGATGCTCTTCGTCCAG (SEQ ID No. 6);
gusB-for: GGAGGTGATTCAGCCACAGC (SEQ ID No. 7);
gusB-rev: TCGGCTTCTGATGCGTCTTA (SEQ ID No. 8).

All QPCR experiments were run on an ABI Prism 7700 Sequence Detector (Applied Biosystems).

The PCR protocol was the following: 50˚C for 2 min hold, 95˚C for 2 min hold, 40 cycles of: 95˚C, 15 sec, 60˚C, 1 min; 95˚C for 15 sec. 20 min-long temperature gradient from 60˚C to 95˚C (melting curve step).

At the end of the run, fluorescence data acquired during PCR were processed as described in the ABI Prism 7700 user's manual.

The melting temperature profile analysis of the PCR products was made using the "Dissociation Curves 1.0" software (Applied Biosystems). No primer-dimers were produced in any of the QPCR experiments.

To calculate the number of copies of *neomycin* and/or *c-Photina*™ gene per diploid genome (i.e., per cell) we entered the Cts (Cycle Threshold) and the PCR efficiencies in the following formula:

$$\text{\# of copies per cell} = 2 \times \frac{(\text{PCR Efficiency }_{target})^{-(Ct\ target)}}{(\text{PCR Efficiency }_{gusB})^{-(Ct\ gusB)}}$$

Ct *gusB* = Ct of the *gusB* gene.

The fraction on the right of the formula gives the number of copies of insert DNA per *gusB* copy. Since two *gusB* copies are present in a diploid genome, the fraction is multiplied by two.

**Southern Blot**

10 μg of ES genomic DNA was digested with different restriction enzymes, HindIII, XbaI, BamHI, HindIII/XbaI (Biolabs), loaded on 0.8% agarose gel, and transferred on a nylon membrane positively charged (Roche, Cat. N. 1417240). As probe was used the [$^{32}$P]dCTP - labelled *c-Photina*™ coding sequence (59).

**P19 cell culture**

P19 Culture medium, seeding and incubation

P19 embryonic carcinoma pluripotent stem cells (ATCC, Cat. N. CRL-1825) are cultured with 10% Foetal Bovine Serum, FBS (ES qualified, Invitrogen, Cat. N. 16141079) αMEM, Minimum Essential Medium Eagle with GLUTAMAX (Invitrogen, Cat. N. 32571028), 1% Pen./Strep. (Invitrogen, Cat. N.15140122) at 37˚C, 5% $CO_2$ (61).

Mito c-Photina™ stable transfection

DNA construct was transfected using electroporation methods that can be replaced with a preferred protocol.

About 10 µg of the *mito c-Photina™* in pcDNA3 DNA was linearized with BglII (New England Biolabs) and was transfected by electroporation in $2.5 \cdot 10^6$ cells (electroporation conditions: 500 µF, 0.24 kV, BioRad gene pulser).

The selection was started after 48 h from the transfection with 700 µg/ml G418.

Colonies were generally ready for picking 8-9 days after electroporation.

Clone Selection Process:

1. P19 pcDNA3/ mito c-Photina™ clones.
2. 24 h and 48 h after seeding, the transfected cells were plated in 2x96MTP white plates at 10000 and 15000 cells/well.
3. Medium was replaced with 50 µl/well of tyrode (2 mM $Ca^{2++}$ and coelenterazine 10 µM).
4. Positive clones were selected evaluating:

- Response to 100 µM Histamine. CCD camera-based luminometer conditions: high sensitivity, reading time 60 seconds.
- Residual total photoprotein activity measured after TRITON X-100 cell lysis. CCD camera-based luminometer conditions: low sensitivity, for 30 seconds.

Transient transfection Analysis:

1. 24 h after transfection medium was replaced with 50 µl/well of tyrode (2 mM $Ca^{2++}$ and coelenterazine 10 µM) and incubated for 4 h at 37˚C.
2. 50 µl/well of 200 µM Histamine (2X) was injected using Luminoskan Ascent (Labsystems). Luminometer conditions: integration time 1 sec, reading time 60 seconds.

- Residual total photoprotein activity was measured after TRITON X-100 cell lysis at Microlumat LB 96P (EG&G Berthold). Reading time 3 seconds.

Mito i-Photina™ and mito Photina™ stable transfection

DNA constructs were transfected using electroporation methods that can be replaced with a preferred protocol.

About 10 µg of the *mito Photina™* and *mito i-Photina™* DNA were linearized with BglII (New England Biolabs) and were transfected by electroporation in $2.5 \cdot 10^6$ cells (electroporation condition: 500 µF, 0.24 kV, BioRad gene pulser).

The selections were started after 48 h from the transfection with 700 µg/ml G418.

Colonies were pooled and collected 9 days after electroporation.

- Response to 50, 100 and 150 µM Histamine. CCD camera-based luminometer conditions: high sensitivity, reading time 60 seconds.
- Residual total photoprotein activity was measured after TRITON X-100 cell lysis. CCD camera-based luminometer conditions: low sensitivity, for 30 seconds.

**Fluorometric Imaging Plate Reader (FLIPR®) measurements**

20000 cells/well were plated in 384 black wall clear bottom plates (MATRIX Cat. N. 4332) (25 µl/well), the tests were run 24 h after cell seeding. Before running experiments the medium was removed, and cells were incubated in 50 µl/well Calcium 3 Assay kit 0.5X (Molecular Devices, Cat. N. R8090) for 30 min at 37˚C. 25 µl/well of Histamine 300 µM (3X) were injected and the fluorescence signal was recorded for 180 sec and expressed as RFU (Relative Fluorescence Units).

FLIPR[384] settings:

Exp. Time: 0.2 sec
Injection speed: 20 $\mu$l/sec
Injection height: 50 $\mu$l

**EXAMPLES**

**1. Generation of ES cell line transfected with photoproteins**

The murine ES TBV2 mito c-Photina™ cell line was obtained by electroporation of ES TBV2 p16 cells with a pcDNA3 vector containing the mito c-Photina™ photoprotein gene linearized with BglII restriction enzyme (Materials and Methods). 48 hours after transfection the cells were put in selection with 200 $\mu$g/ml G418. After 8 days of selection, 152 drug resistant colonies were picked. After morphological analysis only about 114 were expanded on MEF layers till they reach the confluence in 5 replicates in 96 well/plates of which:

> 1- Two were on gelatin coated 96 white MTP for the test at the CCD camera-based luminometer at 24 and 48 h after seeding.
> 2- One was on gelatin coated 96 white MTP for the DNA extraction.
> 3- Two were on feeders layers for freezing and storage at -80˚C.

**2. ES clone selection**

4 hours before measurement the medium of the positive clones was replaced with 50 $\mu$l/well of tyrode buffer 2 mM $Ca^{2+}$ and 10 $\mu$M coelenterazine in the dark, at 37˚C in a 5% $CO_2$ atmosphere, in order to reconstitute the active photoprotein.
For light emission measurement, cells were first analyzed for the ability to respond (luminescent signal) to Histamine which is known to stimulate the ES endogenous Histamine -1 receptor (58) and to rise the cytoplasmic $Ca^{2+}$ concentration. The number of photons emitted after injection of 100 $\mu$M Histamine during the first 60 seconds was measured by a CCD camera-based luminometer. The kinetics of the response obtained is shown in Fig. 1A.
At the end of each experiment, cells were lysed (by a solution containing TRITON X-100). All the photoprotein expressed in the cells react with free calcium and light emitted was measured (Fig.1B). The signal is an indicator of the total amount of photoprotein contained within the cells.
The 12 best Histamine responding clones were selected and retested at counted cells in 96 MTP (Fig. 2A and B).
The two final clones were selected on the basis of different parameters. The ability to respond to Histamine (Fig.3) and the total photoprotein content after cell lysis (data not shown) were the main discriminating factors, but also cell morphology and growth rate were analyzed. Furthermore Southern blot analysis (59) and Quantitative PCR (60) were performed. The Southern blot analysis was fundamental to ensure that only one random insertion occurred. To check this, the genomic DNA was digested with restriction enzymes that cut only one time in the vector transfected (to look for concatenates) or a double digestion with two enzymes (both used also in a single digestion) able to excide the photoprotein gene, assuring the specificity of the results. The probe used for the assay was the photoprotein gene. A quantitative PCR was also performed in order to analyze the number of gene insertion.
Final clones were also characterized by karyotype analysis (61). The clones N. 29 and 84 were selected. The clone 29 has only one photoprotein gene copy integrated in the genome, while the clone 84 has two copies as an inverted concatenate integrated only one time in the genome.
They were also differentiated into spontaneously beating cardiomyocytes after hanging drop Embryoid Bodies formation standard method (57).

**3. Germline transmission analysis**

The mouse embryonic stem cells (ES TBV2) containing the photoprotein reporter gene was tested by germline transmission. Clones N. 29 and 84 were both injected into blastocysts of pregnant host female mice (EMBL Monterotondo). The progenies showed a high degree of chimerism (almost 100%) and male phenotypes. The best male mice, when they reached the sexual maturity, were crossed with BL6 female mice to investigate the germline transmission ability of the transgenic ES cells. The germline transmission is the only incontrovertible way to demonstrate the totipotency of the mouse embryonic stem cells.
The mice born from these crosses are transgenic mice heterozygotic for the photoprotein gene.
They are a very precious source of other cells as the adult stem cells (for example haematopoietic, or mesenchymal stem cells), committed progenitors, and also primary cells.

### 4. Generation of P19 cell line transfected with photoproteins

The P19 mito c-Photina™ cell line was obtained by electroporation of P 19 cells with a pcDNA3 vector containing the *mito c-Photina*™ photoprotein gene linearized with BglII restriction enzyme (Materials and Methods).
48 hours after transfection the cells were put in selection with 700 $\mu$g/ml G418. After about 7-8 days of selection, drug resistant colonies were picked, and expanded.

### 5. P19 selection

4 hours before measurement the medium was replaced with 50 $\mu$l/well of tyrode buffer 2 mM $Ca^{2+}$ and 10 $\mu$M coelenterazine in the dark, at 37˚C in a 5% $CO_2$ atmosphere, in order to reconstitute the active photoprotein.
For light emission measurement, cells were first analyzed for the ability to respond (luminescent signal) to Histamine which is known to stimulate the P19 endogenous Histamine -1 receptor (58) and to rise the cytoplasmic $Ca^{2+}$ concentration.
Two final clones were selected on the basis of the photoprotein activity in response to Histamine and on the photoprotein total content measured after cell lysis with TRITON X-100 (Fig.4). It was also considered their ability to differentiate into spontaneously beating cardiomyocytes and neurons after differentiation process obtained using Embryoid Bodies formation in presence or absence of 0.5-1% DMSO as inducing agent (61,62).

### 6. Transient transfections

The P19 cells were transfected with different mitochondrial tagged photoproteins (materials and methods) to evaluate the ability of these other photoproteins to measure intracellular calcium release and to obtain information on the photoprotein expression levels.
4 hours before measurement the medium was replaced with tyrode buffer and 10 $\mu$M coelenterazine in the dark, at 37˚C in a 5% $CO_2$ atmosphere, in order to reconstitute the active photoprotein.
The luminescence signal was recorded for 60 seconds after 100 $\mu$M Histamine injection.
The cells were then lysed with TRITON X-100 in order to detect the total light release (Fig.5).

### 7. Stable transfections

Stable transfections of the different mitochondrial tagged photoprotein in P 19 cells (materials and methods) were performed in order to investigate the levels of photoprotein expression in a stably integrated manner and to verify that none of the photoproteins stably expressed in P 19 cells are toxic over a long period of time in culture.
4 hours before measurement the medium was replaced with tyrode buffer and 10 $\mu$M coelenterazine in the dark, at 37˚C in a 5% $CO_2$ atmosphere, in order to reconstitute the active photoprotein.
The luminescence signal was recorded after 50, 100 and 150 $\mu$M Histamine injection and measured for 60 seconds.
The cells were then lysed with TRITON X-100 in order to detect the total light release (Fig.6).

### 8. Cell-based fluorescence assays at the FLIPR[384]

The P19 mito c-Photina™ final clones (1A1 and 1A2) were tested also at FLIPR[384] by measuring the calcium concentrations variation induced by the activation of the endogenous Histamine 1 receptor with a detection method that uses fluorescence instead of luminescence.
The cells were incubated with the Calcium 3 assay kit (Molecular Devices
Corporation, Sunnyvale, CA, USA).
These experiments were carried out to compare the results obtained using fluorescence calcium detection methods instead of luminescence-based calcium detection and to evaluate the advantages of luminescence over fluorescence. The results obtained show that fluorescence-based method has a higher background compare to luminescence. This is reflected in a lower signal to noise background of fluorescence. On the contrary, the signal to noise background for luminescence is higher and this reflects in a wider dynamic range compare to fluorescence (Fig.7).

### REFERENCES

1. Burdon T., Smith A., Savatier P. (2002) Signalling, cell cycle and pluripotency in embryonic stem cells. Trends Cell Biol.; 12(9), 432-8.

2. Odorico J.S., Kaufman D.S., Thomson JA. (2001) Stem Cells. Multilineage differentiation from human embryonic stem cell lines.; 19(3), 193-204.

3. Wobus, AM and Boheler, KR. (2005) Embryonic stem cells: prospects for developmental biology and cell therapy. Physiological Reviews; 85, 635-678.

4. Spangrude, G. J., Heimfeld, S. and Weissman, I. L. (1988) Purification and characterization of mouse hematopoietic stem cells. Science; 241, 58-62.

5. Krause D.S., Theise N.D. Collector M.I., Henegariu O., Hwang S., Gardner R., Neutzel S., Sharkis S.J. (2001) Multi-organ, multi-lineage engraftment by a single bone marrow derived stem cell. Cell; 105, 369-377.

6. Reya T., Sean J. Morrison S.J., Clarke M.F.3 and Weissman I.L. (2001) Stem cells, cancer, and cancer stem cells. Nature; 414, 105-111.

7. Wagers A.J, Weissman I.L. (2004) Plasticity of adult stem cells. Cell; 116(5), 639-648.

8. Baksh D., Song L., Tuan R.S. (2004) Adult mesenchymal stem cells: characterization, differentiation, and application in cell and gene therapy. J Cell Mol Med.; 8(3), 301-316.

9. Cai J., Weiss M.L., Rao MS (2004) In search of "stemness". Exp Hematol.; 32(7), 585-598.

10. Donovan P.J., and Gearhart J. (2001) The end of the beginning for pluripotent stem cells. Nature; 414, 92-97.

11. Evans M.J., Kaufman M.H. (1981) Establishment in culture of pluripotential cells from mouse embryos. Nature; 292(5819), 154-156.

12. Martin G.R. (1981) Isolation of a pluripotent cell line from early mouse embryos cultured in medium conditioned by teratocarcinoma stem cells. Proc Natl Acad Sci USA.; 78(12), 7634-7638.

13. Thomson J.A., Itskovitz-Eldor J., Shapiro S.S., Waknitz M.A., Swiergiel J.J., Marshall V.S., Jones J.M. (1998) Embryonic stem cell lines derived from human blastocysts. Science; 282(5391), 1145-1147.

14. Stevens, L. C. (1967) The biology of teratomas. Adv. Morphogen.; 6, 1-31.

15. Gearhart J.D., Mintz B. (1974) Contact-mediated myogenesis and increased acetylcholinesterase activity in primary cultures of mouse teratocarcinoma cells. Proc Natl Acad Sci USA.; 71(5), 1734-1738.

16. Kahan B.W., Ephrussi B.J. (1970) Developmental potentialities of clonal in vitro cultures of mouse testicular teratoma. Natl Cancer Inst.; 44(5),1015-1036.

17. Jiang, L. I. and Nadeau, J. H. (2001) 129/Sv mice--a model system for studying germ cell biology and testicular cancer. Mammal. Genome 12, 89-94.

18. McBurney M.W. (1993) P19 embryonal carcinoma cells. Int J Dev Biol. Mar; 37(1), 135-140.

19. Matsui, Y., Zsebo, K. and Hogan, B. L. (1992) Derivation of pluripotential embryonic stem cells from murine primordial germ cells in culture. Cell; 70, 841-847.

20. Labosky P.A., Barlow D.P., Hogan B.L. (1994a) Embryonic germ cell lines and their derivation from mouse primordial germ cells. Ciba Found Symp.;182,157-68; discussion 168-178.

21. Labosky P.A., Barlow D.P., Hogan B.L. (1994) Mouse embryonic germ (EG) cell lines: transmission through the germline and differences in the methylation imprint of insulin-like growth factor 2 receptor (Igf2r) gene compared with embryonic stem (ES) cell lines.Development; 120(11), 3197-3204.

22. Resnick JL, Bixler LS, Cheng L, Donovan PJ. (1992) Long-term proliferation of mouse primordial germ cells in culture. Nature; 359(6395),550-551.

23. Bradley A., Evans M., Kaufman M.H., and Robertson E. (1984) Formation of germ-line chimeras from embryo-derived teratocarcinoma cell lines. Nature; 309, 255-256.

24. Stewart C.L., Gadi I., and Bhatt H. (1994) Stem cells from primordial germ cells can reenter the germ line. Dev. Biol.; 161, 626-628.

25. Brinster, R.L. (1974) The effect of cells transferred into the mouse blastocyst on subsequent development. J. Exp. Med. 140, 1049-1056.

26. Illmensee K., and Mintz B. (1976) Totipotency and normal differentiation of single teratocarcinoma cells cloned by injection into blastocysts. Proc. Natl Acad. Sci. USA; 73, 549-553.

27. Papaioannou V.E., Gardner R.L., McBurney M.W., Babinet, C. and Evans M.J. (1978) Participation of cultured teratocarcinoma cells in mouse embryogenesis. J. Embryol. Exp. Morphol.; 44, 93-104.

28. Martin G.R. (1980) Teratocarcinomas and mammalian embryogenesis. Science 209, 768-776.

29. McNeish J. (2004) Embryonic stem cells in drug discovery. Nat Rev Drug Discov.; 3(1), 70-80.

30. Seiler A., Visan A., Buesen R., Genschow E. and Spielmann H.(2004) Improvement of an in vitro Stem Cell Assay for developmental toxicity: the use of molecular endpoints in the embryonic stem cell test. Reproductive Toxicology, 18, 231-240.

31. Genschow E., Spielmann H., Scholz G., Seiler A., Brown N., Piersma A., Brady M., Clemann N., Huuskonen H., Paillard F., Bremer S., and Becker K. (2002) The ECVAM international validation study on in vitro embryotoxicity tests: results of the definitive phase and evaluation of prediction models. European Centre for the Validation of Alternative Methods. Altern Lab Anim.; 30(2), 151-76.

32. Genschow E., Spielmann H., Scholz G., Pohl I., Seiler A., Clemann N., Bremer S., and Becker K. (2004) Validation of the embryonic stem cell test in the international ECVAM validation study on three in vitro embryotoxicity tests. Altern Lab Anim.; 32(3), 209-244.

33. Kendall J.M., and Badminton M.N. (1998) Aequorea victoria bioluminescence moves into an exciting new era. Trends Biotechnology; 16(5), 216-224.

34. Campbell A.K., Hallet, R.A., Daw, M.E., Ryall, R.C., Hart, and Herring P.J. (1981) Application of the photoprotein obelin to the measurement of free Ca++ in cells. In Bioluminescence and Chemiluminescence, basic Chemistry and Analytical applications (Edited by M.A. deLuca and W.D. McElroy), pp. 601-607. Academy Press, New York.

35. Herring P.J. (1979) Some features of the bioluminescence of the radiolarian Thalassicola sp. Mar. Biol.; 53, 213-216.

36. Shimomura O., Johnson F.H., and Saiga Y. (1962) Extraction, purification and properties of aequorin, a bioluminescent protein from the luminous hydromedusan, Aequorea. J. Cell. Comp. Physiol.; 59, 223-239.

37. Shimomura O., Johnson F.H., and Saiga, Y (1963) Further data on the bioluminescent protein, aequorin. J. Cell. Comp. Physiol.; 62, 1-8.

38. Morin J.G. and Hastings J.W. (1971) Biochemistry of the bioluminescence of colonial hydroids and other coelenterates. J. Cell. Physiol.; 77, 305-311.

39. Shimomura O., Johnson F.H. and Saiga, Y. (1963) Extraction and properties of halistaurin, a bioluminescent protein from the hydromedusan Halistaura. J. Cell. Physiol.; 62, 9-15.

40. Shimomura O., and Shimomura A. (1985) Halistaurin, phialidin and modified forms of aequorin as Ca++ indicator in biological systems. Biochem. J.; 228, 745-749.

41. Levine L.D., and Ward W.W. (1982) Isolation and characterization of a photoprotein "phialidin" and a spectrally unique green-fluorescent protein from the bioluminescent jellyfish Phialidium gregarium. Comp. Biochem. Physiol.; 72B, 77-85.

42. Morin J.G. and Hastings J.W. (1971) Energy transfer in a bioluminescent system. J. Cell. Physiol. 77, 313-318.

43. Campbell A.K. (1974) Extraction, partial purification and properties of obelin the calcium-activated protein from the hydroid Obelia geniculata. Biochem.J.; 143, 411-418.

44. Ward W.W. and Selinger H.H. (1974) Extraction and purification of calcium-activated photoprotein from the ctenophores Mnemiopsis sp. and Bern ovata. Biochemistry; 13, 1491-1499.

45. Ward W.W. and Seliger H.H. (1974) Properties of mnemiopsin, and berovin, calcium-activated photoproteins from the ctenophores Mnemiopsis sp. and Beroë ovata. Biochemistry 13, 1500-1510.

46. Johnson F.H. and Shimomura O. (1978) Introduction to the bioluminescence of medusae, with special reference to the photoprotein aequorin. Methods Enzymol.; 57, 271-291.

47. Illarionov B.A., Bondar V.S., Illarionova V.A., Vysotski E.S. (1995) Sequence of the cDNA encoding the Ca++-activated photoprotein obelin from the hydroid polyp Obelia longissima. Gene; 14;153(2), 273-274.

48. Blinks J.R., Weir W.G., Hess P. and Prendergast F.G. (1982) Measurement of Ca++ concentrations in living cells. Prog. Biophys. Mol. Biol.; 40,1-114.

49. Markova S.V., Vysotski E.S., Blinks J.R., Burakova L.P., Wang B.C., Lee J., (2002) Obelin from the bioluminescent marine hydroid Obelia geniculata: cloning, expression, and comparison of some properties with those of other Ca2+-regulated photoproteins. Biochemistry; 41 (7),2227-36.

50. Inouye S., Tsuji F.I. (1993) Cloning and sequence analysis of cDNA for the Ca(2+)-activated photoprotein, clytin. FEBS Lett.; 315(3), 343-346.

51.

Tsuji F.I., Ohmiya Y., Fagan T.F., Toh H., Inouye S. (1995) Molecular evolution of the Ca(2+)-binding photoproteins of the Hydrozoa. Photochem Photobiol.; 62(4), 657-661.

52. Rizzuto R., Simpson A.W.M., Brini M. and Pozzan T. (1992) Rapid changes of mitochondrial Ca2+ revealed by specifically targeted recombinant aequorin. Nature; 358, 325-328.

53. Rizzuto R., Brini M., Murgia M. and Pozzan T. (1993) Microdomains with high Ca2+ close to IP3-sensitive channels that are sensed by neighbouring mitochondria. Science; 262, 744-747.

54. Rizzuto R., Bastianutto C., Brini M., Murgia M. and Pozzan T. (1994) Mitochondrial Ca2+ homeostasis in intact cells. J. Cell Biol.; 126, 1183-1194.

55. Nagy A., Gertsenstein M., Vinterstein K., Behringer R. (2003) Manipulating the mouse embryo. A laboratory manual. Third edition. Cold Spring Harbor Laboratory Press.

56. Turksen K.(2002) Embryonic Stem Cells. Methods and protocols. Methods in Molecular Biology. Vol 185. Humana Press

57. Boheler K.R. (2003) ES cell differentiation to the cardiac lineage. Methods in enzymology; 365, 228-241.

58. Bloemers S.M., Leurs R., Smit M.J., Verheule S., Tertoolen L.G.J., Timmerman H., and de Laat S.W. (1993) Mouse P19 embryonal carcinoma cells express functional Histamine H1-receptors. Biochemical and Biophysical Research Communications; 191, 118-125.

59. Sambrook J., and Russel D. W. (2001) Molecular cloning. A laboratory manual. Third edition. Cold Spring Harbor Laboratory Press.

60. Ramakers C., Ruijter J.M., Deprez R.H., Moorman A.F. (2003) Assumption-free analysis of quantitative real-time polymerase chain reaction (PCR) data. Neuroscience Letters; 339, 62-66.

61. Rudnicki M.A. and McBurney M.W. (1987) Cell culture methods and induction of differentiation of embryonal carcinoma cell lines. In: E.J. Robertson (Ed.), Teratocarcinomas and Embryonic Stem Cells: A Practical Approach, IRL Press, Oxford, pp. 19-49.

62. J. Paquin B. Danalache M. Jankowski S.M., McCann J., Gutkowska J. (2002) Oxytocin induces differentiation of P19 embryonic stem cells to cardiomyocytes. Proc. Nat. Acad. Sci. USA; 99, 9550-9555.

63. Bolino A., Bolis A., Previtali S.C., Dina G., Bussini S., Dati G., Amadio S., Del Carro U., Mruk D.D., Feltri M.L., Cheng C.Y., Quattrini A., Wrabetz L. (2004). Disruption of Mtmr2 produces CMT4B1-like neuropathy with myelin outfolding and impaired spermatogenesis. J Cell Biol; 167, 711-721.

**Claims**

1. A stable recombinant stem cell able to express an apophotoprotein and produce a bioluminescent signal in the presence of a suitable chromophore as substrate in response to intracellular calcium concentration variation.

2. The stable recombinant stem cell according to claim 1 being a non human totipotent or pluripotent cell; a human or non-human pluripotent tumoral cell or a multipotent cell or a progenitor thereof, being of embryonic or of adult origin.

3. The stable recombinant stem cell according to claim 1 or 2 wherein the apophotoprotein gene is any apophotoprotein gene, of natural or recombinant or synthetic origin.

4. The stable recombinant stem cell according to claim 3 wherein the apophotoprotein is a natural or mutagenized mutant having an improved luminescent activity and/or calcium sensibility.

5. The stable recombinant stem cell according to claim 3 or 4 wherein the apophotoprotein gene has a sequence optimized for mammalian codon usage and/or fused to mitochondrial target sequences.

6. The stable recombinant stem cell according to any of previous claims wherein the apophotoprotein sequence is as SEQ ID No. 1.

7. The stable recombinant stem cell according to any of previous claims 1-5 wherein the apophotoprotein sequence is the Clytin sequence (GenBank accession number Q08121) mutagenised in the following position $Gly_{142} \rightarrow Cys$.

8. The stable recombinant stem cell according to any of previous claims 1-5 wherein the apophotoprotein sequence is the Clytin sequence (GenBank accession number Q08121) mutagenised in the following 12 positions: $Gly_{58} \rightarrow Glu$, $Asp_{69} \rightarrow Val$, $Ala_{70} \rightarrow Cys$, $Lys_{76} \rightarrow Arg$, $Lys_{77} \rightarrow Gly$, $Ile_{78} \rightarrow Cys$, $Asp_{81} \rightarrow Glu$, $Val_{86} \rightarrow Ile$, $Glu_{87} \rightarrow Ala$, $Ala_{90} \rightarrow Gln$, $Val_{92} \rightarrow Leu$, and $Glu_{97} \rightarrow Gln$.

9. The stable recombinant stem cell according to any of previous claims being differentiated into a specific cell lineage to get expression of a target.

10. The stable recombinant stem cell according to claim 9 wherein the specific cell lineage is the muscle heart cell lineage or the neuronal lineage or the mesenchimal cell lineage.

11. A non human transgenic mammal and cell derivatives thereof able to express an apophotoprotein gene and to produce a bioluminescent signal in response to intracellular calcium concentration variation.

12. The non human transgenic mammal and cell derivatives thereof according to claim 11 wherein the apophotoprotein gene expression and/or the production of a bioluminescent signal in response to intracellular calcium concentration variation is ubiquitous.

13. The non human transgenic mammal and cell derivatives thereof according to claim 11 wherein the apophotoprotein gene expression and/or the production of a bioluminescent signal in response to intracellular calcium concentration variation is organ-, tissue-, cell- or development stage-specific.

14. A method for identifying agents stimulating the differentiation of stem cells towards a specific cell lineage comprising the steps of:

    a) providing stable recombinant stem cells according to claims 1-8 at an undifferentiated stage;
    b) exposing said cells to a compound library comprising putative inducing differentiation agents to get expression of at least one specific cell lineage target;
    c) loading cells with a suitable chromophore as substrate;
    d) stimulating said specific cell lineage target by a ligand so that a variation of intracellular $Ca^{++}$ is obtained;
    e) detecting photoprotein's bioluminescence.

15. The method according to claim 14 wherein the specific cell lineage is the muscle heart cell lineage or the neuronal lineage.

**16.** The method according to claim 14 or 15 being performed by High Throughput Screening.

**17.** A method for identifying agents inhibiting the differentiation of stem cells towards a specific cell lineage comprising the steps of:

a) providing stable recombinant stem cells according to claims 1-8 at an undifferentiated stage;
b) exposing said cells to a compound library comprising putative inhibiting differentiation agents;
c) exposing said cells to a known inducing differentiation agent to get expression of at least one specific cell lineage target;
d) loading cells with a suitable chromophore as substrate;
e) stimulating said specific cell lineage target by a ligand so that a variation of intracellular $Ca^{++}$ is obtained;
f) detecting photoprotein's bioluminescence.

**18.** The method according to claim 17 wherein the specific cell lineage is the muscle heart cell lineage or the neuronal lineage.

**19.** The method according to claims 17 or 18 being performed by High Throughput Screening.

**20.** A method for identifying a ligand able to stimulate a specific target so that a variation of intracellular $Ca^{++}$ is obtained comprising the steps of:

a) providing stable recombinant stem cells according to claims 1-8;
b) differentiating said cells into a specific cell lineage to get expression of the target;
c) loading cells with a suitable chromophore as substrate;
d) contacting cells with a compound library comprising putative ligands for said target;
e) detecting the photoprotein's bioluminescence.

**21.** The method according to claim 20 wherein the specific cell lineage is the muscle heart cell lineage or the neuronal lineage.

**22.** The method according to claims 20 or 21 being performed by High Throughput Screening.

**23.** A method for identifying antagonists to a target, so that a variation of intracellular $Ca^{++}$ is obtained, comprising the steps of:

a) providing stable recombinant stem cells according to claims 1-8;
b) differentiating said cells into a specific cell lineage to get expression of said target;
c) loading cells with a suitable chromophore as substrate;
d) contacting cells with a compound library comprising putative antagonists for said target;
e) contacting cells with a ligand able to stimulate the said target;
f) detecting the photoprotein's bioluminescence variation.

**24.** The method according to claim 23 wherein the specific cell lineage is the muscle heart cell lineage or the neuronal lineage.

**25.** The method according to claims 23 or 24 being performed by High Throughput Screening.

**26.** Use of the stable recombinant stem cells according to claims 1-10 for *in vitro* testing of toxicity and/or teratology of a substance.

FIG. 1 A

**ES / mito c-Photina™ clones**
**96 MTP**
**100 μM Histamine**

FIG. 1 B

ES / mito c-Photina™ clones
96 MTP
Total light release upon cell lysis

**FIG. 2A**

ES / mito c-Photina™ 12 best clones
96 MTP – 20000 cells /well
100 µM Histamine

**FIG. 2B**

ES / mito c-Photina™ 12 best clones
96 MTP
Total light release upon cell lysis

FIG. 3A

**ES / mito c-Photina™ 2 final clones**
**96 MTP – 10000 cells /well**
**100 µM Histamine**

FIG. 3B

**ES / mito c-Photina™ 2 final clones**
**96 MTP – 20000 cells /well**
**100 µM Histamine**

FIG. 4A

**P19 / mito c-Photina™ 1A1 clone**
**96 MTP – 20000 cells /well**
**Histamine response**

FIG. 4B

**P19 / mito c-Photina™ 1A2 clone**
**96 MTP – 20000 cells /well**
**Histamine response**

FIG. 4C

P19 / mito c-Photina™ 2 final clones
96 MTP
Total light release upon cell lysis

FIG. 5A

**P19 / mito photoprotein transient transfection
96 MTP – 5000 cells /well
100 µM Histamine**

FIG. 5B

**P19 / mito photoprotein transient transfection
96 MTP – 5000 cells /well
Total light release upon cell lysis**

FIG. 6A

P19 / mito i-Photina™ pool stable transfection
96 MTP – 10000 cells /well
Histamine response

FIG. 6B

P19 / mito Photina™ pool stable transfection
96 MTP – 10000 cells /well
Histamine response

FIG. 6C

**P19 / mito photoprotein pool stable transfection**
**96 MTP – 10000 and 20000 cells /well**
**Total light release upon cell lysis**

FIG. 7A

**P19 / mito c-Photina™ 1A1 clone**
**384 MTP - 20000 cells/well**

|  | signal/noise |
|---|---|
| fluorescence | 1 |
| luminescence | 4 |

FIG. 7B

**European Patent Office**

# EUROPEAN SEARCH REPORT

Application Number

EP 06 00 0452

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2004/035620 A (AXXAM S.R.L; FOTI, MARIA; LOHMER, STEFAN) 29 April 2004 (2004-04-29) * page 3, line 28 - page 8, line 15 * ----- | 1-10, 14-26 | INV. C07K14/435 C12N15/12 G01N33/68 |
| Y | WO 98/35022 A (OSIRIS THERAPEUTICS, INC) 13 August 1998 (1998-08-13) * page 3, paragraph 4 * ----- | 1-10, 14-26 | |
| X | WO 01/18225 A (XENOGEN CORPORATION) 15 March 2001 (2001-03-15) * abstract * ----- | 11-13 | |
| A | CHIESA A ET AL: "RECOMBINANT AEQUORIN AND GREEN FLUORESCENT PROTEIN AS VALUABLE TOOLS IN THE STUDY OF CELL SIGNALLING" BIOCHEMICAL JOURNAL, THE BIOCHEMICAL SOCIETY, LONDON, GB, vol. 355, no. 1, 1 April 2001 (2001-04-01), pages 1-12, XP009013205 ISSN: 0264-6021 * the whole document * ----- | 1 | TECHNICAL FIELDS SEARCHED (IPC) C07K |
| A | US 5 714 666 A (PRITCHETT, DECEASED ET AL) 3 February 1998 (1998-02-03) * the whole document * ----- | 1 | |

The present search report has been drawn up for all claims

| Place of search | Date of completion of the search | Examiner |
|---|---|---|
| Munich | 5 July 2006 | Nichogiannopoulou, A |

EPO FORM 1503 03.82 (P04C01)

European Patent

Office

Application Number

EP 06 00 0452

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

```
see sheet B
```

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

| | | |
|---|---|---|
| **European Patent**<br>**Office** | **LACK OF UNITY OF INVENTION**<br>**SHEET B** | Application Number<br>EP 06 00 0452 |

The Search Division considers that the present European patent application does not comply with the requirements of unity of invention and relates to several inventions or groups of inventions, namely:

```
1. claims: 1-10 and 14-26

        Recombinant stem cells, methods and uses relating to the
        expression of an apophotoprotein in stem cells capable of
        producing a bioluminescent signal in response to
        intracellular calcium concentration variation.
                          ---

2. claims: 11-13

        Non-human ransgenic mammals expressing an apophotoprotein
        and producing a bioluminescent signal in response to
        intracellular calcium concentration variation.
                          ---
```

## ANNEX TO THE EUROPEAN SEARCH REPORT
## ON EUROPEAN PATENT APPLICATION NO.

EP 06 00 0452

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2006

| Patent document cited in search report | | Publication date | Patent family member(s) | | Publication date |
|---|---|---|---|---|---|
| WO 2004035620 | A | 29-04-2004 | AT | 290016 T | 15-03-2005 |
| | | | AU | 2003287962 A1 | 04-05-2004 |
| | | | BR | 0306589 A | 30-11-2004 |
| | | | CA | 2503145 A1 | 29-04-2004 |
| | | | CN | 1705680 A | 07-12-2005 |
| | | | DE | 60203123 D1 | 07-04-2005 |
| | | | DE | 60203123 T2 | 21-07-2005 |
| | | | DK | 1413584 T3 | 04-07-2005 |
| | | | EP | 1413584 A1 | 28-04-2004 |
| | | | ES | 2237643 T3 | 01-08-2005 |
| | | | HR | 20050354 A2 | 31-08-2005 |
| | | | MX | PA05004148 A | 03-08-2005 |
| WO 9835022 | A | 13-08-1998 | AU | 6144498 A | 26-08-1998 |
| WO 0118225 | A | 15-03-2001 | AU | 2589500 A | 10-04-2001 |
| US 5714666 | A | 03-02-1998 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1413584 A
- EP 05005390 A
- EP 06000171 A

### Non-patent literature cited in the description

- **BURDON T. ; SMITH A. ; SAVATIER P.** Signalling, cell cycle and pluripotency in embryonic stem cells. *Trends Cell Biol.,* 2002, vol. 12 (9), 432-8
- **ODORICO J.S. ; KAUFMAN D.S. ; THOMSON JA.** Multilineage differentiation from human embryonic stem cell lines. *Stem Cells,* 2001, vol. 19 (3), 193-204
- **WOBUS, AM ; BOHELER, KR.** Embryonic stem cells: prospects for developmental biology and cell therapy. *Physiological Reviews,* 2005, vol. 85, 635-678
- **SPANGRUDE, G. J. ; HEIMFELD, S. ; WEISSMAN, I. L.** Purification and characterization of mouse hematopoietic stem cells. *Science,* 1988, vol. 241, 58-62
- **KRAUSE D.S. ; THEISE N.D. ; COLLECTOR M.I. ; HENEGARIU O ; HWANG S. ; GARDNER R. ; NEUTZEL S. ; SHARKIS S.J.** Multi-organ, multi-lineage engraftment by a single bone marrow derived stem cell. *Cell,* 2001, vol. 105, 369-377
- **REYA T. ; SEAN J. ; MORRISON S.J. ; CLARKE M.F. ; WEISSMAN I.L.** Stem cells, cancer, and cancer stem cells. *Nature,* 2001, vol. 414, 105-111
- **WAGERS A.J ; WEISSMAN I.L.** Plasticity of adult stem cells. *Cell,* 2004, vol. 116 (5), 639-648
- **BAKSH D. ; SONG L. ; TUAN R.S.** Adult mesenchymal stem cells: characterization, differentiation, and application in cell and gene therapy. *J Cell Mol Med.,* 2004, vol. 8 (3), 301-316
- **CAI J. ; WEISS M.L. ; RAO MS.** In search of ''stemness. *Exp Hematol.,* 2004, vol. 32 (7), 585-598
- **DONOVAN P.J. ; GEARHART J.** The end of the beginning for pluripotent stem cells. *Nature,* 2001, vol. 414, 92-97
- **EVANS M.J. ; KAUFMAN M.H.** Establishment in culture of pluripotential cells from mouse embryos. *Nature,* 1981, vol. 292 (5819), 154-156
- **MARTIN G.R.** Isolation of a pluripotent cell line from early mouse embryos cultured in medium conditioned by teratocarcinoma stem cells. *Proc Natl Acad Sci USA.,* 1981, vol. 78 (12), 7634-7638

- **THOMSON J.A. ; ITSKOVITZ-ELDOR J. ; SHAPIRO S.S. ; WAKNITZ M.A. ; SWIERGIEL J.J. ; MARSHALL V.S. ; JONES J.M.** Embryonic stem cell lines derived from human blastocysts. *Science,* 1998, vol. 282 (5391), 1145-1147
- **STEVENS, L. C.** The biology of teratomas. *Adv. Morphogen.,* 1967, vol. 6, 1-31
- **GEARHART J.D. ; MINTZ B.** Contact-mediated myogenesis and increased acetylcholinesterase activity in primary cultures of mouse teratocarcinoma cells. *Proc Natl Acad Sci USA.,* 1974, vol. 71 (5), 1734-1738
- **KAHAN B.W. ; EPHRUSSI B.J.** Developmental potentialities of clonal in vitro cultures of mouse testicular teratoma. *Natl Cancer Inst.,* 1970, vol. 44 (5), 1015-1036
- **JIANG, L. I. ; NADEAU, J. H.** 129/Sv mice--a model system for studying germ cell biology and testicular cancer. *Mammal. Genome,* 2001, vol. 12, 89-94
- **MCBURNEY M.W.** P19 embryonal carcinoma cells. *Int J Dev Biol. Mar,* 1993, vol. 37 (1), 135-140
- **MATSUI, Y. ; ZSEBO, K. ; HOGAN, B. L.** Derivation of pluripotential embryonic stem cells from murine primordial germ cells in culture. *Cell,* 1992, vol. 70, 841-847
- **LABOSKY P.A. ; BARLOW D.P. ; HOGAN B.L.** Embryonic germ cell lines and their derivation from mouse primordial germ cells. *Ciba Found Symp.,* 1994, vol. 182, 157-68
- **LABOSKY P.A. ; BARLOW D.P. ; HOGAN B.L.** Mouse embryonic germ (EG) cell lines: transmission through the germline and differences in the methylation imprint of insulin-like growth factor 2 receptor (Igf2r) gene compared with embryonic stem (ES) cell lines. *Development,* 1994, vol. 120 (11), 3197-3204
- **RESNICK JL ; BIXLER LS ; CHENG L ; DONOVAN PJ.** Long-term proliferation of mouse primordial germ cells in culture. *Nature,* 1992, vol. 359 (6395), 550-551

- **BRADLEY A. ; EVANS M. ; KAUFMAN M.H. ; ROBERTSON E.** Formation of germ-line chimeras from embryo-derived teratocarcinoma cell lines. *Nature,* 1984, vol. 309, 255-256
- **STEWART C.L. ; GADI I. ; BHATT H.** Stem cells from primordial germ cells can reenter the germ line. *Dev. Biol.,* 1994, vol. 161, 626-628
- **BRINSTER, R.L.** The effect of cells transferred into the mouse blastocyst on subsequent development. *J. Exp. Med.,* 1974, vol. 140, 1049-1056
- **ILLMENSEE K. ; MINTZ B.** Totipotency and normal differentiation of single teratocarcinoma cells cloned by injection into blastocysts. *Proc. Natl Acad. Sci. USA,* 1976, vol. 73, 549-553
- **PAPAIOANNOU V.E. ; GARDNER R.L. ; MCBURNEY M.W. ; BABINET, C. ; EVANS M.J.** Participation of cultured teratocarcinoma cells in mouse embryogenesis. *J. Embryol. Exp. Morphol.,* 1978, vol. 44, 93-104
- **MARTIN G.R.** Teratocarcinomas and mammalian embryogenesis. *Science,* 1980, vol. 209, 768-776
- **MCNEISH J.** Embryonic stem cells in drug discovery. *Nat Rev Drug Discov.,* 2004, vol. 3 (1), 70-80
- **SEILER A. ; VISAN A. ; BUESEN R. ; GENSCHOW E. ; SPIELMANN H.** Improvement of an in vitro Stem Cell Assay for developmental toxicity: the use of molecular endpoints in the embryonic stem cell test. *Reproductive Toxicology,* 2004, vol. 18, 231-240
- **GENSCHOW E. ; SPIELMANN H. ; SCHOLZ G. ; SEILER A. ; BROWN N. ; PIERSMA A. ; BRADY M. ; CLEMANN N. ; HUUSKONEN H. ; PAILLARD F.** The ECVAM international validation study on in vitro embryotoxicity tests: results of the definitive phase and evaluation of prediction models. *European Centre for the Validation of Alternative Methods. Altern Lab Anim.,* 2002, vol. 30 (2), 151-76
- **GENSCHOW E. ; SPIELMANN H. ; SCHOLZ G. ; POHL I. ; SEILER A. ; CLEMANN N. ; BREMER S. ; BECKER K.** Validation of the embryonic stem cell test in the international ECVAM validation study on three in vitro embryotoxicity tests. *Altern Lab Anim.,* 2004, vol. 32 (3), 209-244
- **KENDALL J.M. ; BADMINTON M.N.** Aequorea victoria bioluminescence moves into an exciting new era. *Trends Biotechnology,* 1998, vol. 16 (5), 216-224
- Application of the photoprotein obelin to the measurement of free Ca++ in cells. **CAMPBELL A.K. ; HALLET, R.A. ; DAW, M.E. ; RYALL, R.C. ; HART ; HERRING P.J.** Bioluminescence and Chemiluminescence, basic Chemistry and Analytical applications. Academy Press, 1981, 601-607
- **HERRING P.J.** Some features of the bioluminescence of the radiolarian Thalassicola sp. *Mar. Biol.,* 1979, vol. 53, 213-216
- **SHIMOMURA O. ; JOHNSON F.H. ; SAIGA Y.** Extraction, purification and properties of aequorin, a bioluminescent protein from the luminous hydromedusan, Aequorea. *J. Cell. Comp. Physiol.,* 1962, vol. 59, 223-239
- **SHIMOMURA O. ; JOHNSON F.H. ; SAIGA, Y.** Further data on the bioluminescent protein, aequorin. *J. Cell. Comp. Physiol.,* 1963, vol. 62, 1-8
- **MORIN J.G. ; HASTINGS J.W.** Biochemistry of the bioluminescence of colonial hydroids and other coelenterates. *J. Cell. Physiol.,* 1971, vol. 77, 305-311
- **SHIMOMURA O. ; JOHNSON F.H. ; SAIGA, Y.** Extraction and properties of halistaurin, a bioluminescent protein from the hydromedusan Halistaura. *J. Cell. Physiol.,* 1963, vol. 62, 9-15
- **SHIMOMURA O. ; SHIMOMURA A.** Halistaurin, phialidin and modified forms of aequorin as Ca++ indicator in biological systems. *Biochem. J.,* 1985, vol. 228, 745-749
- **LEVINE L.D. ; WARD W.W.** Isolation and characterization of a photoprotein ''phialidin'' and a spectrally unique green-fluorescent protein from the bioluminescent jellyfish Phialidium gregarium. *Comp. Biochem. Physiol.,* 1982, vol. 72B, 77-85
- **MORIN J.G. ; HASTINGS J.W.** Energy transfer in a bioluminescent system. *J. Cell. Physiol.,* 1971, vol. 77, 313-318
- **CAMPBELL A.K.** Extraction, partial purification and properties of obelin the calcium-activated protein from the hydroid Obelia geniculata. *Biochem.J.,* 1974, vol. 143, 411-418
- **WARD W.W. ; SELINGER H.H.** Extraction and purification of calcium-activated photoprotein from the ctenophores Mnemiopsis sp. and Bern ovata. *Biochemistry,* 1974, vol. 13, 1491-1499
- **WARD W.W. ; SELIGER H.H.** Properties of mnemiopsin, and berovin, calcium-activated photoproteins from the ctenophores Mnemiopsis sp. and Beroë ovata. *Biochemistry,* 1974, vol. 13, 1500-1510
- **JOHNSON F.H. ; SHIMOMURA O.** Introduction to the bioluminescence of medusae, with special reference to the photoprotein aequorin. *Methods Enzymol.,* 1978, vol. 57, 271-291
- **ILLARIONOV B.A. ; BONDAR V.S. ; ILLARIONOVA V.A. ; VYSOTSKI E.S.** Sequence of the cDNA encoding the Ca++-activated photoprotein obelin from the hydroid polyp Obelia longissima. *Gene,* 1995, vol. 153 (2), 273-274
- **BLINKS J.R. ; WEIR W.G. ; HESS P. ; PRENDERGAST F.G.** Measurement of Ca++ concentrations in living cells. *Prog. Biophys. Mol. Biol.,* 1982, vol. 40, 1-114

- **MARKOVA S.V. ; VYSOTSKI E.S. ; BLINKS J.R. ; BURAKOVA L.P. ; WANG B.C. ; LEE J.** Obelin from the bioluminescent marine hydroid Obelia geniculata: cloning, expression, and comparison of some properties with those of other Ca2+-regulated photoproteins. *Biochemistry,* 2002, vol. 41 (7), 2227-36
- **INOUYE S. ; TSUJI F.I.** Cloning and sequence analysis of cDNA for the Ca(2+)-activated photoprotein, clytin. *FEBS Lett.,* 1993, vol. 315 (3), 343-346
- **TSUJI F.I. ; OHMIYA Y. ; FAGAN T.F. ; TOH H. ; INOUYE S.** Molecular evolution of the Ca(2+)-binding photoproteins of the Hydrozoa. *Photochem Photobiol.,* 1995, vol. 62 (4), 657-661
- **RIZZUTO R. ; SIMPSON A.W.M. ; BRINI M. ; POZZAN T.** Rapid changes of mitochondrial Ca2+ revealed by specifically targeted recombinant aequorin. *Nature,* 1992, vol. 358, 325-328
- **RIZZUTO R. ; BRINI M. ; MURGIA M. ; POZZAN T.** Microdomains with high Ca2+ close to IP3-sensitive channels that are sensed by neighbouring mitochondria. *Science,* 1993, vol. 262, 744-747
- **RIZZUTO R. ; BASTIANUTTO C. ; BRINI M. ; MURGIA M. ; POZZAN T.** Mitochondrial Ca2+ homeostasis in intact cells. *J. Cell Biol.,* 1994, vol. 126, 1183-1194
- Manipulating the mouse embryo. **NAGY A. ; GERTSENSTEIN M. ; VINTERSTEIN K. ; BEHRINGER R.** A laboratory manual. Cold Spring Harbor Laboratory Press, 2003
- Embryonic Stem Cells. **TURKSEN K.** Methods in Molecular Biology. Humana Press, 2002, vol. 185
- **BOHELER K.R.** ES cell differentiation to the cardiac lineage. *Methods in enzymology,* 2003, vol. 365, 228-241
- **BLOEMERS S.M. ; LEURS R. ; SMIT M.J. ; VERHEULE S. ; TERTOOLEN L.G.J. ; TIMMERMAN H. ; DE LAAT S.W.** Mouse P19 embryonal carcinoma cells express functional Histamine H1-receptors. *Biochemical and Biophysical Research Communications,* 1993, vol. 191, 118-125
- **SAMBROOK J. ; RUSSEL D. W.** Molecular cloning. A laboratory manual. Cold Spring Harbor Laboratory Press, 2001
- **RAMAKERS C. ; RUIJTER J.M. ; DEPREZ R.H. ; MOORMAN A.F.** Assumption-free analysis of quantitative real-time polymerase chain reaction (PCR) data. *Neuroscience Letters,* 2003, vol. 339, 62-66
- Cell culture methods and induction of differentiation of embryonal carcinoma cell lines. **RUDNICKI M.A. ; MCBURNEY M.W.** Teratocarcinomas and Embryonic Stem Cells: A Practical Approach. IRL Press, 1987, 19-49
- **J. PAQUIN B. ; DANALACHE M. ; JANKOWSKI S.M. ; MCCANN J. ; GUTKOWSKA J.** Oxytocin induces differentiation of P19 embryonic stem cells to cardiomyocytes. *Proc. Nat. Acad. Sci. USA,* 2002, vol. 99, 9550-9555
- **BOLINO A. ; BOLIS A. ; PREVITALI S.C. ; DINA G. ; BUSSINI S. ; DATI G. ; AMADIO S. ; DEL CARRO U. ; MRUK D.D. ; FELTRI M.L.** Disruption of Mtmr2 produces CMT4B1-like neuropathy with myelin outfolding and impaired spermatogenesis. *J Cell Biol,* 2004, vol. 167, 711-721

SEQUENCE LISTING

<110>  AXXAM s.r.l.

<120>  LUMINESCENT STEM CELLS, TRANSGENIC MAMMALS AND USES THEREOF

<130>  BE 89996

<160>  8

<170>  PatentIn version 3.3

<210>  1
<211>  195
<212>  PRT
<213>  chimeric protein

<400>  1

Met Ser Ser Lys Tyr Ala Val Lys Leu Lys Thr Asp Phe Asp Asn Pro
1               5                   10                  15

Arg Trp Ile Lys Arg His Lys His Met Phe Asp Phe Leu Asp Ile Asn
                20                  25                  30

Gly Asn Gly Lys Ile Thr Leu Asp Glu Ile Val Ser Lys Ala Ser Asp
            35                  40                  45

Asp Ile Cys Ala Lys Leu Gly Ala Thr Pro Glu Gln Thr Lys Arg His
        50                  55                  60

Gln Asp Ala Val Glu Ala Phe Phe Lys Lys Ile Gly Met Asp Tyr Gly
65                  70                  75                  80

Lys Glu Val Glu Phe Pro Ala Phe Val Asp Gly Trp Lys Glu Leu Ala
                    85                  90                  95

Thr Ser Glu Leu Lys Lys Trp Ala Arg Asn Glu Pro Thr Leu Ile Arg
                100                 105                 110

Glu Trp Gly Asp Ala Val Phe Asp Ile Phe Asp Lys Asp Gly Ser Gly
            115                 120                 125

Thr Ile Thr Leu Asp Glu Trp Lys Ala Tyr Gly Lys Ile Ser Gly Ile
        130                 135                 140

Ser Pro Ser Gln Glu Asp Cys Glu Ala Thr Phe Arg His Cys Asp Leu
145                 150                 155                 160

Asp Asn Ser Gly Asp Leu Asp Val Asp Glu Met Thr Arg Gln His Leu
                165                 170                 175

Gly Phe Trp Tyr Thr Leu Asp Pro Glu Ala Asp Gly Leu Tyr Gly Asn
                180                 185                 190

Gly Val Pro

195

```
<210>    2
<211>    99
<212>    DNA
<213>    Homo sapiens

<400>    2
atgtccgtcc tgacgccgct gctgctgcgg ggcttgacag gctcggcccg gcggctccca        60

gtgccgcgcg ccaagatcca ttcgttggga tccgccacc                                99


<210>    3
<211>    19
<212>    DNA
<213>    synthetic primer

<400>    3
caccaagtgt gcgtggagg                                                      19


<210>    4
<211>    20
<212>    DNA
<213>    synthetic primer

<400>    4
gcgatctcct tgccgtactc                                                     20


<210>    5
<211>    20
<212>    DNA
<213>    synthetic primer

<400>    5
cacgtactcg gatggaagcc                                                     20


<210>    6
<211>    20
<212>    DNA
<213>    synthetic primer

<400>    6
ccctgatgct cttcgtccag                                                     20


<210>    7
<211>    20
<212>    DNA
<213>    synthetic primer

<400>    7
ggaggtgatt cagccacagc                                                     20


<210>    8
<211>    20
<212>    DNA
<213>    synthetic primer

<400>    8
tcggcttctg atgcgtctta                                                     20
```